# EUROPEAN PATENT APPLICATION

(11) **EP 0 753 312 A1**
(43) Date of publication of application: **15.01.1997**
(21) Application number: 94907700.2
(22) Date of filing: 28.02.1994
(51) Int. Cl.: A61K 48/00, A61K 31/70, C12N 15/12, A61K 38/00, C12N 15/85

(54) **ARTERIOSCLEROSIS REMEDY**

(30) Priority: 29.09.1993 JP 243381/93
(71) Applicant: KIRIN BEER KABUSHIKI KAISHA, Chuo-Ku, Tokyo 104 (JP); OSAKA PREFECTURAL GOVERNMENT, Osaka-shi, Osaka 540 (JP)
(72) Inventor: TAKAHASHI, Katsuhito, Ikeda-shi, Osaka 563 (JP); SHIBATA, Nobuhiko, Sakai-shi, Osaka 593 (JP)
(74) Representative: Hansen, Bernd, Dr. Dipl.-Chem.
(86) International application number: JP9400320
(87) International publication number: WO9509010

(57) **Abstract**

An arteriosclerosis remedy containing a carponin gene as the active ingredient, which is useful because it can inhibit the thickening of the endangium. It is therefore efficacious for the treatment of arteriosclerosis and ischemic heart diseases which may be caused thereby, such as angina pectoris and myocardial infarction. In particular, it can remarkably effectively inhibit the occurrence of restenosis after percutaneous transluminal coronary angioplasty (PTCA).

## Description

### TECHNICAL FIELD

The present invention relates to therapeutic agents for arteriosclerosis, more specifically to therapeutic agents for arteriosclerosis utilizing a calponin gene.

### BACKGROUND OF THE INVENTION

Arteriosclerosis is pathologically represented by an intimal lesion characterized by the deposition of lipid and connective tissue and the growth of smooth muscle cells and macrophages. In particular, atherosclerosis causes ischemic heart diseases and cerebral hemorrhage and is, therefore, a very fatal disease. It is generally considered that hyperlipidemia, hypertension, aging and smoking are the major risk factors of arteriolerosis.

For the treatment of arteriosclerosis, antihyperlipidemia agents and anti-hypertensive drugs are used as therapeutic agents for depressing enhancement of the risk factors. Antioxidants, antiplatelet agents, vasodilators, anticoagulants and the like are used as therapeutic agents for suppressing the causes of the onset of the disease. At present, these treatments have not produced clinically sufficient effects.

In addition, percutaneous transluminal coronary angioplasty (PTCA) is performed in the early treatment of ischemic heart diseases such as angina pectoris and cardiac infarction caused by arteriosclerosis. The PTCA produces significant results. However, it is a great problem that restenosis is found in 30-40 % of patients three to six months after PTCA treatment. A drug is administered to patients for suppressing restenosis but this treatment is not sufficiently effective. The results of various studies show that smooth muscle cell-proliferation in the intima causes restenosis (Hanke, H. et al., Time course of smooth muscle cell proliferation in the intima and media of arteries of following experimental angioplasty, Circulation Res. 1990; 67: 651-659). A method of treating restenosis by directly introducing an agent or gene into the intimal smooth muscle cells specifically at the diseased sites has been tried. As one example, a method of applying heparin on the intimal smooth muscle cells specifically at the diseased sites was tried (Gimple, L. W. et al., Effect of chronic subcutaneous or intramural administration of heparin on femoral artery restenosis after balloon angioplasty in hypercholesterolemic rabbits. Circulation 1992; 86: 1536-1546) but did not produce sufficient effects to suppress restenosis. Furthermore, a trial of introducing a luciferase encoding gene into smooth muscle cells specifically at the positions of arteriosclerotic lesion formed by ballooning was made (Leclerc, G. et al., Percutaneous arterial gene transfer in a rabbit model. J. Clin. Invest. 1992; 90: 936-944) but this trial was not completely satisfactory as a treatment of arteiosclerosis, because they used this gene not as therapy but as marker.

Accordingly, an object of the present invention is to provide therapeutic agents for arteriosclerosis which can overcome the above mentioned disadvantages of the prior art. In other words, an object of the present invention is to provide highly effective therapeutic agents for arteriosclerosis.

Another object of the present invention is to provide therapeutic agents for arteriosclerosis which can prevent effectively restenosis after PTCA.

A further object of the present invention is to provide recombinant vectors which are useful for preparing the highly effective therapeutic agents for arteriosclerosis.

A still object of the present invention is to provide pharmaceutical compositions which are useful for treating arteriosclerosis and an ischemic heart disease it induces.

An additional object of the present invention is to provide a method of treating arteriosclerosis.

An even further object of the present invention is to provide a method of treating an ischemic heart disease.

### DISCLOSURE OF THE INVENTION

As a result of various studies, the inventors have found that smooth muscle cell proliferation in the walls of blood vessels could be suppressed by introducing calponin gene into the wall of blood vessel and have accomplished the present invention. The present invention provides therapeutic agents for arteriosclerosis, which comprise a calponin gene as an active ingredient. Furthermore, the present invention provides recombinant vectors that contain a calponin gene and which are capable of expressing the calponin gene in a smooth muscle cell. The present invention also provides pharmaceutical compositions comprising a calponin gene and a pharmaceutically acceptable carrier. In addition, the present invention provides a method of treating arteriosclerosis, comprising the step of administering to a patient a therapeutically effective amount of calponin gene. In addition, the present invention provides a method of treating an ischemic heart disease comprising the step of administering to a patient a therapeutically effective amount of a calponin gene.

While not being limited by a particular theory, it is believed that by the application of the therapeutic agents for arteriosclerosis of the present invention, the calponin gene is expressed in a smooth muscle cell in the wall of a blood vessel and that whereby the dedifferentiated smooth muscle cell is redifferentiated to suppress the smooth muscle cell proliferation.

### BRIEF DESCRIPTION OF THE DRAWINGS

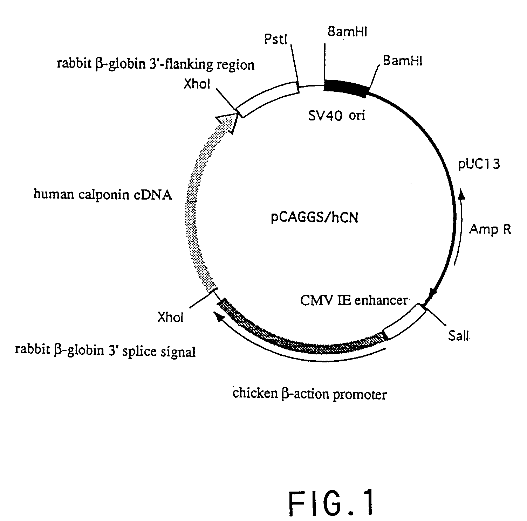

Figure 1 is a restriction map of the recombinant vector pCAGGS/hCN incorporating the human calponin cDNA.

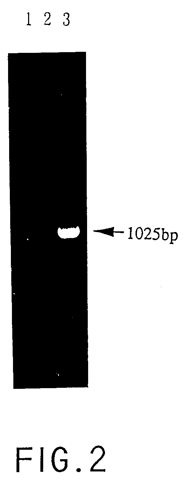

Figure 2 is an electropherogram for confirming the introduction of the human calponin gene in the wall of a blood vessel. Lane 1 is a negative control containing no DNA, lane 2 is the DNA from the common carotid artery of the control group 2 treated with plasmid pCAGGS and lane 3 is the DNA from the common carotid artery of the calponin group treated with the recombinant vector pCAGGS/hCN containing the human calponin cDNA.

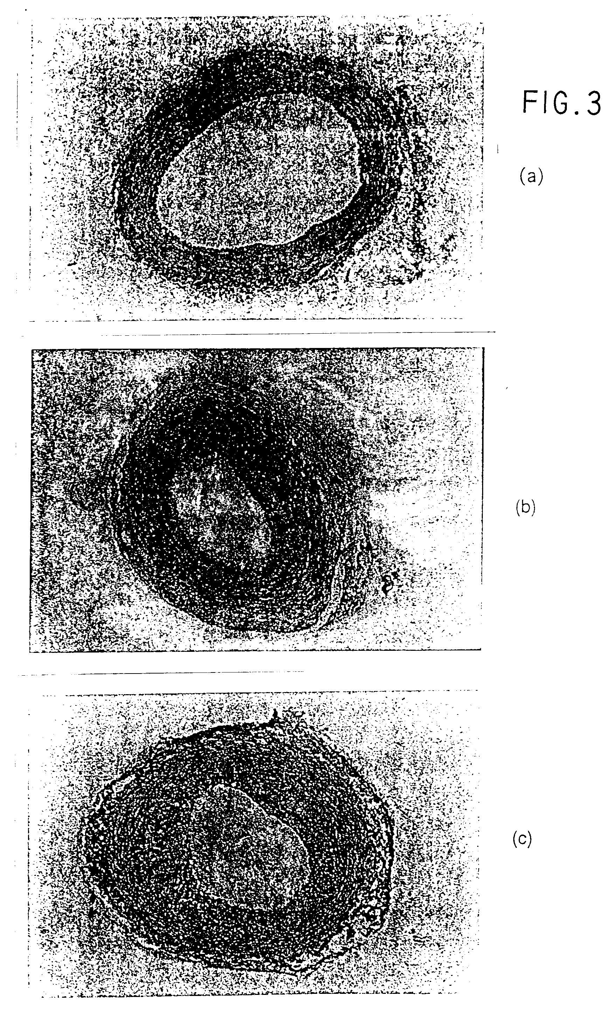

Figure 3 is a set of photographs showing the biological morphology of the hyperplastic intima of a blood vessel observed in a cross section of the common carotid artery of the following tree groups: (a) the calponin group treated with the recombinant vector pCAGGS/hCN containing the human calponin cDNA; (b) the control group 2 treated with plasmid pCAGGS; and (c) the non-treated control group 1.

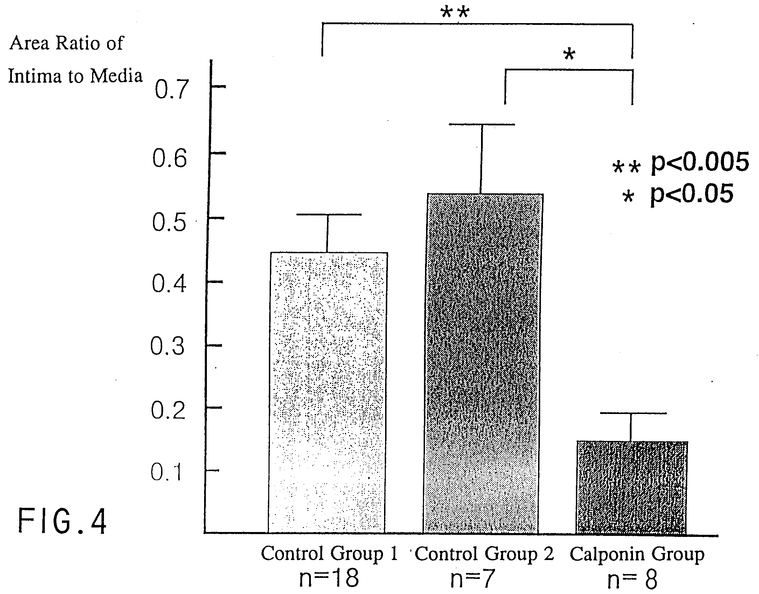

Figure 4 is a graph showing how the introduction of gene after rubbing the rabbit common carotid artery with a balloon was effective in suppressing the the intimal hyperplasia of blood vessel(s) wall. On the abscissa of Figure 4, control group 1 represents the non-treated control group subjected to no treatment after being rubbed in the common carotid artery using the balloon (n=18), control group 2 represents the control group treated with plasmid pCAGGS after being rubbed in the common carotid artery using the balloon (n=7) and calponin group represents the group treated with the recombinant vector pCAGGS/hCN containing the human calponin cDNA (n=8). The numbers on the ordinate indicate the area ratio of the intima to the media.

### BEST MODE FOR CARRYING OUT THE INVENTION

The term "calponin gene" means a gene encoding calponin or a calponin-like protein. Calponin was found as a troponin-like protein present mainly in mammalian smooth muscle cells (Takahashi, K. et al., Biochem. Biophys. Res. Commun. 1986, 141: 20-26). It is known that calponin exists on actin filaments and inhibits the activity of myosin ATPase by bonding to actin filaments (Winder, S. J. et al., J. Biol. Chem. 1990; 265: 10148-10155). Therefore, calponin is believed to play an important role in regulating the contraction of smooth muscle. The amino acid sequence of chicken calponin was determined by Takahashi et al (Takahashi, K. and Nadal-Ginard, B., J. Biol. Chem. 1991; 266: 13284-13288). SM22 and mp20 are known as calponin-like proteins and the amino acid sequences of them were determined by Thweatt and Ayme-Southgate, respectively (Thweatt, R. et al., Biochem. Biophys. Res. Commun. 1992; 187: 1-7 and Ayme-Southgate, A. et al., J. Cell. Biol. 1989; 108: 521-531). The aforementioned calponin and calponin-like proteins can be used in the present invention.

The cDNA of calponin was first cloned from a chicken gizzard and the base sequence thereof was determined (Takahashi, K. and Nadal-Ginard, B., J. Biol. Chem. 1991; 266: 13284-13288). Thereafter, the cloning of human and rat calponin cDNAs was reported (Takahashi, K. et al., Japanese Circulation Journal 1992; 56 supplement 1: 40 and Shanahan, C. M. et al., Circulation Res. 1993; 73: 193-204). Such calponin cDNAs can be used in the present invention. The calponin gene to be introduced is preferably derived from man for minimizing the immunorejection of the introduced gene and the expressed protein and for increasing the effectiveness of the treatment. The calponin gene containing the base sequence encoding the amino acid sequence of SEQ ID No: 2 is preferably used and the calponin gene containing the base sequence of SEQ ID No: 1 is more preferably used. The calponin gene may contain supplementary coding sequences. The calponin gene can be modified so as to add, replace or delete some of the encoded amino acid sequence, provided that it can express a protein having a function similar to that of calponin. The calponin gene may be a gemonic DNA or cDNA obtained by isolation and purification from cells using conventional techniques, or it may be chemically synthesized according to the method of Narang et al (Narang, S. A., DNA synthesis. Tetrahedron 1983; 39: 3).

The calponin gene per se can be introduced into smooth muscle cells at the diseased sites. Alternatively, a recombinant vector that contains the calponin gene and which is capable of expressing the calponin gene in smooth muscle cells can be introduced into a smooth muscle cell at the diseased site. Such a recombinant vector comprises the calponin gene and an expression vector for expressing it. In general, the expression vector can be constructed with a plasmid vector or viral vector DNA or RNA which are capable of expressing proteins in mammalian cells. The expression vector can be constructed by optimally selecting and combining a replication origin which renders an expression vector replicable, a promoter for expression, a splice signal, poly A addition signal, drug selection marker, an enhancer and the like. Preferably, the expression vector contains at least a promoter. Examples of the replication origin which renders an expression vector replicable include SV40 virus, papilloma virus, EB virus (Epstein-Barr virus) and the like. Examples of the promoters for expression include β-actin promoter, elongation factor 1 α, thymidine kinase promoter, SV40 promoter, adenovirus major late promoter, cytomegalovirus, promoter and the like. Furthermore, a transcriptional control gene which controls the expression of an introduced gene may be added, in addition to a splice signal, poly A addition signal, a drug selection marker for assuring efficient clone selection, such as ampicillin-resistance gene, and a cell-specifically functioning enhancer. Examples of the preferred expression vectors that may be used to construct the recombinant vector of the present invention include pEF-BOS (Mizushima, S. et al., Nucleic Acid Research 1990; 18: 5322), pcDL-SR α 296 (Takebe, Y. et al., Molecular and Cellular Biology 1988; 8: 466-472), pCAGGS (Niwa, H. et al., Efficient selection for high-expression transfectants with a novel eukaryotic vector, Gene 1991; 108: 193-200), pAd265SVp(A)3 (Kaufman, R. J., et al., Mol. Cell. Biol. 1985; 5: 1750-1759) and the like. Among them, pCAGGS is most preferred. pCAGGS which contains the enhancer of cytomegalovirus, chicken β-actin promoter, rabbit β-globin 3' splice signal, rabbit β-globin 3'-flanking region and SV40 replication origin is capable of efficient expression of a gene incorporated into mammalian cells.

The recombinant vector of the present invention can be constructed by incorporating the calponin gene in the expression vector using techniques well known to a skilled person in the art (Maniatis, T. et al., Molecular cloning: A laboratory manual 1989; Cold spring harbor laboratory).

The calponin gene, or the recombinant vector that contains the calponin gene and which is capable of expressing the calponin gene in smooth muscle cells (hereinafter referred to as "the calponin gene-containing recombinant vector") may be formulated in a pharmaceutically acceptable carrier to prepare a pharmaceutical composition in the form of a solution, suspension, gel or the like for subsequent administration.

Alternatively, the calponin gene or the calponin gene-containing recombinant vector may be encapsulated in a membrane to prepare a particle and the particle may be formulated in a pharmaceutical acceptable carrier to prepare a pharmaceutical composition in the form of a solution, suspension, gel or the like for subsequent administration. The encapsulation of the calponin gene or the calponin gene-containing recombinant vector in a membrane can prevent the gene or recombinant vector from being digested with nuclease and can facilitate the highly efficient introduction of the gene or recombinant vector into a target cell without injury. As the membrane, synthetic membranes such as liposome (Wong, T. K. et al., Science 1980; 215: 166), lipid emulsions and the like and naturally occurring membranes such as protoplast (Schaffner, W., Proc. Natl. Acad. Sci. 1980; 77: 2163), capsids of virus such as retrovirus (Cone, R.D. et al. , Proc. Natl. Acad. Sci. 1984; 81: 6349), erythrocyte ghost (Furusawa, M. et al., Nature 1974; 2498: 449) and the like can be used. Among them, liposome is preferred for the following reasons. The calponin gene or the calponin gene-containing recombinant vector per se can be encapsulated in liposome without subjecting the gene or recombinant vector to any special treatment. In addition, if liposome is produced from the lipid that is present in humans or the lipid that is metabolized in the human body, the liposome is metabolized to become a harmless form after the introduction. Examples of materials used to form liposome include lipids such as N-[1-(2,3-dioleoyloxy)-propyl]-N,N,N-trimethyl ammonium methyl sulfate (DOTAP), N-[1-(2,3-dioleyloxy)-propyl]-N,N,N-trimethyl ammonium chloride (DOTMA), dilauroyl phosphatidylcholine (DLPC), dioleoyl phosphatidylethanol amine (DOPE), dilauroyl phosphatidylethanol amine (DLPE), dimyristoyl phosphatidylethanol amine (DMPE), dioleoyl phosphatidylcholine (DOPC), dimyristoyl phosphatidylcholine (DMPC), N-(α-trimethyl ammonioacetyl)-didodecyl-D-glutamate chloride (TMAG) and the like, as well as mixtures thereof. These lipids are preferred since they can form a large unilamellar vesicle (LUV) having a sufficient internal volume to permit efficient incorporation of the calponin gene or the calponin gene-containing recombinant vector without injuring these DNAs. For example, it is known that a mixture of N-[1-(2,3-dioleyloxy)-propyl]-N,N,N-trimethyl ammonium chloride (DOTMA) with dioleoyl phosphatidylethanol amine (DOPE) in a ratio of 1:1 (w/w) (Felgner, D. L. et al, Lipofection: A highly efficient, lipid-mediated DNA-transfection procedure, Proc. Natl. Acad. Sci. 1987; 84: 7413), a mixture of N-(α-trimethyl ammonioacetyl)-didodecyl-D-glutamate chloride (TMAG) with dilauroyl phosphatidylcholine (DLPC) and dioleoyl phosphatidylethanol amine (DOPE) in a ratio of 1:2:2 (mol/mol/mol) (Koshizaka, T. et al., J. Clin. Biochem. Nutr. 1989; 7: 185) and the like form liposomes capable of DNA uptake. Among these lipids, N-[1-(2,3-dioleoyloxy)-propyl]-N,N,N-trimethyl ammonium methyl sulfate (DOTAP) is most preferred because of low cytotoxity. Furthermore, the glycoprotein of HVJ (Hemagglutinating virus of Japan) may be incorporated in or covalently bonded to the surface of liposome, or ethylene glycol may be added in order to increase the efficiency of DNA introduction into a cell. Moreover, an antibody specific to an antigen present on the surface of a target smooth muscle cell or a receptor ligand may be incorporated in or covalently bonded to liposome in order to enhance the targeting specificity for the cell.

The calponin gene or the calponin gene-containing recombinant vector can be encapsulated in the various membranes mentioned above using techniques well known to a skilled person in the art. For example, the calponin gene or the calponin gene-containing recombinant vector can be encapsulated in viral capsids according to the method described in Danos O. et al., Proc. Natl. Acad. Sci. 1988; 85: 6460. The calponin gene or the calponin gene-containing recombinant vector can be encapsulated in synthetic membranes by mixing the calponin gene or the calponin gene-containing recombinant vector with a lipid of the type described above and a liquid medium such as water, Hepes buffered physiological salt solution (150mM NaCl/20mM Hepes, pH 7.4), Tris-HCl buffer and the like and stirring the mixture. When the calponin gene or the calponin gene-containing recombinant vector is to be encapsulated in synthetic membranes, polyethylene glycol, plant lectin and the like may be added. The ratio of the calponin gene to the membrane or the ratio of the calponin gene-containing recombinant vector to the membrane may be selected at such a value as to express the calponin gene in a desired amount. The calponin gene or the calponin gene-containing recombinant vector is preferably contained in the membrane in a proportion of from 10 to 50% by weight.

The calponin gene or the calponin gene-containing recombinant vector which are optionally encapsulated can be administered by various methods such as intravenous injection. Preferably, they are locally administered using a catheter. With the aid of a catheter, the calponin gene can be transferred on or around the diseased sites and introduced at the diseased sites within the wall of a blood vessel at high efficiency by using a catheter. Examples of representative catheters include a single balloon catheter, Wolinsky coronary infusion catheter, a double balloon catheter and the like. As a method of administration using a double balloon catheter, the method of Chang et al (Chang, S. et al., Direct in vivo gene transfer into the coronary and peripheral vasculatures of the intact dog, Circulation, 1991; 83: 2007-2011) can be used.

The calponin gene or the calponin gene-containing recombinant vector as an active ingredient can be formulated together with a pharmaceutically acceptable carrier to prepare a pharmaceutical composition according to conventional formulation techniques. Examples of the pharmaceutically acceptable carrier include diluents, fillers, sterile aqueous media and various non-toxic organic solvents. Pharmaceutically acceptable materials such as stablizers, buffers, isotonizers and the like can be suitably combined or selected for addition to the pharmaceutical composition. Exemplary stabilizers include saccharides such as glucose, mannitol and the like, amino acids such as glycine and the like, HSA, BSA, gelatin, and the like. Exemplary buffers include Tris buffer, PBS buffer, citrate buffer solution, acetate buffer solution, HEPES buffer and the like. Exemplary isotonizers include salts such as sodium chloride and the like, saccharides such as glucose, mannitol and the like, etc. The pharmaceutical preparation produced by solving or suspending the active ingredient in an aqueous solution of the above materials can be used. For increasing the efficiency of gene introduction, polyethylene glycol, DMSO and the like may be added.

When the pharmaceutical preparation comprising the calponin gene or the calponin gene-containing recombinant vector in a non-encapsulated state is to be used, the calponin gene can be introduced in a smooth muscle cell at the diseased site by applying the pharmaceutical preparation on the diseased site within the wall of a blood vessel by calcium phosphate coprecipitation (Graham, F. L. et al., Virology 1973; 52: 456), in vivo transfection using the DEAE dextran procedure (McCutchan, J. H. et al., J. Natl. Cancer Inst. 1968; 41: 351), by ballooning with a single balloon catheter coated with a mixture of the calponin gene or the calponin gene-containing recombinant vector and hydrogel, or the like. When the pharmaceutical preparation comprising the calponin gene or the calponin gene-containing recombinant vector in an encapsulated state is to be used, the calponin gene can be introduced in a smooth muscle cell at the diseased site by application of the pharmaceutical preparation to the diseased site by injecting and incubating the pharmaceutical preparation with a double balloon catheter for a certain period of time or by injecting it into the wall of a blood vessel using a Wolinsky coronary infusion catheter.

The dosage ranges for the administration of the therapeutic agent for arteriosclerosis of the present invention may be varied depending on age, sex, the severity of the disease in the patient, the route of administration, the number of administrations, the dosage form and the like. In general, doses corresponding to the range from about 20 µg to 600 mg of the calponin gene per day is suitable for adults.

The therapeutic agents for arteriosclerosis of the present invention are believed to have low toxicity or high degree of safety since the active ingredient thereof is the gene encoding the calponin present in mammalian smooth muscle cells. In addition, the therapeutic agents for arteriosclerosis of the present invention are believed to have extremely low carcinogenicity since it is known that the introduction of calponin gene into cultured smooth muscle cells suppresses the expression of protooncogene such as c-fos. Moreover, the cytotoxicity of the therapeutic agents for arteriosclerosis of the present invention can be further reduced by encapsulating the calponin gene or the calponin gene-containing recombinant vector in membranes such as liposome and the like.

The present invention will be explained in greater detail with reference to the following examples, which are intended to be illustrative but are not to limit the scope of the present invention.

### Example 1

### 1. Cloning of human calponin cDNA

RNA was isolated from the aorta of a Japanese liver cancer patient (male, 54 years old) and purified according to the method of Chirgwin et al (Chirgwin. J. M. et al., Biochemistry 1977; 18: 5294-5299). poly (A)⁺ RNA was purified from the RNA using Oligotex™-dT30 〈Super〉 (catalog No. 9021B, TAKARA SHUZO CO.,LTD.). A human aorta λ ZAP^{R}-cDNA library was prepared from the poly (A)⁺ RNA using a ZAP-cDNA synthesis kit (catalog No. 200400, Stratagene Co.) and Gigapack^{R} II Gold (catalog No. 200216, Stratagene Co.). A recombinant DNA from this library was plated on a nylon membrane filter Hybond™-N+ (catalog No. RPN.137B, Amersham Co.) and plaque hybridization was performed using chicken calponin cDNA as a probe (Takahashi, K. and Nadal-Ginard, B., J. Biol. Chem. 1991; 266: 13284-13288) to produce positive clones. The positive clones were subcloned in pBluescript SK- by infection with f1 helper phage R408 VCSM13. The longest clone was selected from the subclones and designated pBluescript SK-hCN (Takahashi, K. et al., Japanese Circulation Journal 1992; 56 supplement 1: 40). The pBluescript SK-hCN was sequenced using a Sequenase^{R} Version 2.0 DNA sequencing kit (catalog No. 70781, USB Co.). The base sequence of the human calponin cDNA thus produced is shown under SEQ ID No:1 in SEQUENCE LISTING.

### 2. Construction of a recombinant vector containing the calponin gene

The pBluescript SK-hCN prepared in 1. was linearized with restriction enzyme SmaI (Boehringer Mannheim Co.) and ligated to annealed XhoI linker (Boehringer Mannheim Co.) with T4 DNA ligase (TAKARA SHUZO CO.,LTD.) in order to insert XhoI restriction site at the 5' end of the human calponin cDNA in the pBluescript SK-hCN. E. coli DH5 α cell line (available from Lifetechnologies Inc.) was transformed with the above reaction solution. A plasmid was isolated from the culture of the transformed cells. The isolated plasmid was digested with restriction enzyme XhoI (Boehringer Mannheim Co.) to produce a fragment containing 1522 bp of the human calponin cDNA and a fragment of pBluescript SK- plasmid. This result shows that the purified plasmid was the plasmid of interest.

Then, the plasmid was digested with restriction enzyme XhoI and subjected to 0.8 % agarose gel electrophoresis to isolate the fragment containing 1522 bp of the human calponin cDNA shown under SEQ ID No: 1. This fragment was purified using a Prep-A-Gene DNA purification kit (catalog No. 732-6010, Biorad Co.). The purified DNA fragment was ligated with T4 DNA ligase to plasmid pCAGGS that had been digested with restriction enzyme XhoI (Niwa, H. et al., Gene 1991; 108: 193-200). E. coli DH5 α cell line was transformed with the reaction solution. A plasmid was purified from the culture of the transformed cells and digested with restriction enzymes XhoI and PstI (Boehringer Mannheim Co.) to confirm the insertion of the human calponin cDNA and its direction with respect to the promoter. Since the digestion with XhoI produced a fragment containing 1522 bp of the human calponin cDNA and the digestion with PstI produced a fragment of about 1270 bp containing the 3' end of the human calponin cDNA and rabbit β-globin 3'-flanking region, the purified plasmid was verified as plasmid pCAGGS/hCN which was the recombinant vector for expression of human calponin.

Figure 1 shows a restriction map of the recombinant vector pCAGGS/hCN containing the human calponin cDNA. The size of the recombinant vector pCAGGS/hCN is about 6.5 kb.

### 3. Encapsulation in liposome

N-[1-(2,3-dioleoyloxy)-propyl]-N,N,N-trimethyl ammonium methyl sulfate (DOTAP) (Boehringer Mannheim Co.) (1mg/ml, 60 µl) was diluted with 190 µl of Hepes buffered physiological salt solution (Hepes, 20 mM; NaCl, 150 mM; pH 7.4). In a separate step, the recombinant vector pCAGGS/hCN (30 µg) constructed in 2. was dissolved in 250 µl of Hepes buffered physiological salt solution. The above two solutions were mixed and incubated for at least 20 minutes at room temperature to produce particles having pCAGGS/hCN encapsulated in liposome.

As a control, the above procedure was repeated except that the recombinant vector pCAGGS/hCN constructed in 2. was replaced by plasmid pCAGGS to produce particles having plasmid pCAGGS encapsulated in liposome.

### 4. Preparation of a therapeutic agent for arteriosclerosis

Trypan blue (10 µl) (Sigma Co.) and a physiological salt solution (300 µl) were added to the solution (500 µl) containing the particles having pCAGGS/hCN encapsulated in liposome (prepared in 3.) and mixed to prepare 800 µl of the solution. This solution was used as a therapeutic agent for arteriosclerosis.

The above procedure was repeated except that plasmid pCAGGS was used instead of the recombinant vector pCAGGS/hCN to prepare a control solution.

### 5. Percutaneous transluminal gene transfer (PTGT)

Using eighteen rabbits, the inner part of common carotid artery of each rabbit was rubbed over 4-5 cm at 5 atmospheres using an inflated balloon catheter for PTCA (C.R. Bard Inc.). This operation was repeated 3 times. After 2 days, a 22G cannula (Medikit Co.) was inserted in the ear vein of each of the treated rabbits and 4-5 ml of a solution of Ravonal 1A (TANABE SEIYAKU CO.,LTD.) (thiopental sodium 0.3 g, sodium carbonate 0.18 g) in 30 ml of physiological salt solution was injected intravenously at low speed to anesthetize the rabbit. Thereafter, the rabbits were fixed on benches.

The solution containing the particles having the recombinant vector pCAGGS/hCN encapsulated in liposome was administered to each rabbit using a Wolinsky coronary infusion catheter (C.R. Bard Inc.) by percutaneous transluminal gene transfer (PTGT). Briefly, the inguinal region of each rabbit whose inner part of common carotid artery had been rubbed was incised and the femoral artery was exposed. The rabbit was laid on the back and fixed with a thread. The femoral artery of the rabbit was perforated using a cannula. A 2cm-long Wolinsky coronary infusion catheter (C.R. Bard Inc.) was equipped with a guide wire for PTCA (C.R. Bard Inc.). The common carotid artery preliminarily rubbed with the balloon was selected under X-ray fluoroscopic observation and the guide wire was advanced to the most distal point of the rubbed sites and the Wolinsky coronary infusion catheter was positioned 1 cm proximal to the furcation of the internal carotid artery and the external carotid artery.

Then, an 18F needle (TERUMO CORP.) was equipped with a 30 cm-long pressure tube (catalog No. 9070112, NAMIC Co.) and 800 µl of the encapsulated pCAGGS/hCN solution prepared in 4. was sucked into the pressure tube. The pressure tube was connected to the inlet of the Wolinsky coronary infusion catheter and the encapsulated pCAGGS/hCN solution was injected into 2 cm distal part of the common carotid artery rubbed 2 days before while the pressure was maintained at 8 atmospheres by a Basix ™ Indeflator (MERIT Medical Co.); the injection was made through 10 cycles for about 8 seconds. The guide wire and Wolinsky coronary infusion catheter were removed from the common carotid artery. A solution of antibiotic Cefotax (CHUGAI PHARMACEUTICAL CO.,LTD) (0.5 g) in physiological salt solution (20 ml) was prepared. Three milliters of the Cefotax solution was locally injected in the incised and perforated sites and the remaining Cefotax solution was injected intravenously.

The above procedure was repeated except that the control solution containing the encapsulated plasmid pCAGGS prepared in 4. was used instead of the solution of the encapsulated recombinant vector pCAGGS/hCN (control group 2).

As a control, 2-3 cm proximal part of the common carotid artery rubbed 2 days before was left with no treatment (control group 1).

### 6. Confirmation of the intimal hyperplasia of blood vessels

Fourteen days after the percutaneous transluminal gene transfer (PTGT), the common carotid artery was removed from each of the rabbits rubbed with the balloon. A blood vessel with a length of about 4 mm was set up in Tissue Tek cryomold (available from Miles Laboratory Co.) and a frozen block was prepared using Tissue Tek OCT Compound 4583 (available from Miles Laboratory Co.). The frozen block was cut into slices in a thickness of 5-6 µm using a cryostat. After being air-dried, these slices were subjected to hematoxylin and eosin stain. The degree of the intimal hyperplasia of blood vessels in the three groups, the calponin group treated with the recombinant vector containing the human calponin cDNA (n=8), the control group 1 subjected to no treatment (n=18) and the control group 2 treated with plasmid pCAGGS (n=7), was evaluated with micrographs of cross sections of the common carotid artery wall. Three micrographs of such cross sections are shown in Figure 3. Figure 3 (a) illustrates the morphology of the hyperplastic intima of blood vessel observed in a cross section of the common carotid artery in the calponin group; Figure 3 (b) in the control group 2; and Figure 3 (c) in the control group 1. Figure 3 reveals that the degree of the intimal hyperplasia of blood vessels in the calponin group was smaller than those of the control group 1 and the control group 2.

For numerically expressing the degree of the intimal hyperplasia of blood vessels, the areas of the intima and the media were measured with a planimeter and the area ratio of the intima to the media was calculated as the degree of intimal hyperplasia (Nabel, E.G. et al., J. Clin. Invest. 1993; 91: 1822-1829). The results are shown in Figure 4. On the abscissa of Figure 4, "control group 1" represents the non-treated control group which was given no treatment after being rubbed in the common carotid artery with the balloon (n=18), "control group 2" represents the control group treated with plasmid pCAGGS after being rubbed in the common carotid artery with the balloon (n=7), and "calponin group" represents the group treated with the recombinant vector pCAGGS/hCN containing the human calponin cDNA (n=8). The numbers on the ordinate indicate the area ratio of the intima to the media in cross sections of the common carotid artery. In Figure 4, * and * * indicate significance levels (p) of not more than 0.05 % and 0.005 %, respectively. It was confirmed that the degree of the intimal hyperplasia in the calponin group was significantly smaller than those of the control group 1 and the control group 2.

Therefore, the results of the above experiments demonstrate that the therapeutic agents for arteriosclerosis of the present invention are effective in preventing restenosis after PTCA.

### 7. Confirmation of the introduction of the human calponin gene in the wall of blood vessels

The blood vessel preparations of the common carotid artery removed from the rabbits of the calponin group treated with the recombinant vector pCAGGS/hCN containing the human calponin cDNA (n=8) and those of the control group 2 treated with plasmid pCAGGS (n=7) were subjected to the following treatments.

To the blood vessel preparation of the common carotid artery removed in 6., phosphate buffered saline (PBS) (8 g/l NaCl, 0.2 g/l KCl, 1.15 g/l Na₂HPO₄, 0.2 g/l KH₂PO₄ ) was added in an amount 3 times the weight of said preparation. The preparation was sliced and homogenized. The homogenate was centrifuged at 15,000 rpm for 3 minutes to produce the supernatant. DNA was extracted from the supernatant using a Sepa Gene (catalog No. SG-0025, Sanko Junyaku Co.). The DNA (10 µg) was treated with XhoI (Boehringer Mannheim Co.) and extracted with a mixture of phenol and chloroform. After the DNA was precipitated with ethanol, the resulting precipitate was dissolved in 20 µl of water and the concentration of the DNA was determined in terms of the absorbance at a wavelength of 260 nm.

Polymerase chain reaction was performed using as a template the DNA contained in the solution equivalent to a DNA content of 2 µg. The primers were the following two synthetic oligonucleotides containing the base sequence present in 5' and 3'non-translational regions of human calponin cDNA. For PCR, GeneAmp™ PCR Reagent Kit with AmpliTaq™ DNA Polymerase (catalog No. PJ5100, TAKARA SHUZO CO.,LTD.) was employed under the denaturing condition of 94 °C for 1 minute, the annealing condition of 55°C for 2 minutes and the synthesis condition of 72°C for 2 minutes. The amplification of the DNA fragment (1052 bp) between the above primers specific to human calponin was confirmed by 1.2% agarose gel electrophoresis.
5'GAGTGTGCAGACGGAACTTCAGCC 3' (SEQ ID No:3 : sense sequence of sequence 18-41 under SEQ ID No: 1)
5'GGCTGGGCCTGGCTGGGTCCAGCC 3' (SEQ ID No:4 : antisense sequence of sequence 1046-1069 under SEQ ID No: 1)

The results are shown in Figure 2. In Figure 2, lane 1 is a negative control containing no DNA, lane 2 is the DNA from the common carotid artery of the control group 2 treated with plasmid pCAGGS and lane 3 is the DNA from the common carotid artery of the calponin group treated with the recombinant vector pCAGGS/hCN containing the human calponin cDNA. The DNA fragment with a length of 1052 bp was detected in lane 3 but not in lane 2. These results reveal that the human calponin cDNA gene was introduced only in the calponin group.

### INDUSTRIAL APPLICABILITY

The therapeutic agents for arteriosclerosis of the present invention are useful for suppressing the intimal hyperplasia of blood vessels. Hence, the therapeutic agents for arteriosclerosis of the present invention are effective against arteriosclerosis and ischemic heart diseases it induces, such as angina pectoris and cardiac infarction and, in particular, they are very effective in preventing restenosis after PTCA.

SEQUENCE LISTING

INFORMATION ON SEQ ID NO:1

(i) SEQUENCE CHARACTERISTICS:

(A) LENGTH: 1522 base pairs

(B) TYPE: nucleic acid

(C) STRANDNESS: double

(D) TOPOLOGY: linear

(ii) MOLECULE TYPE: cDNA to mRNA

(iv) ORIGINAL SOURCE: human

(xi) SEQUENCE DESCRIPTION: SEQ ID NO:1:

AACATGTGAG GAGGGAAGAG TGTGCAGACG GAACTTCAGC CGCTGCCTCT GTTCTCAGCG 60

TCAGTGCCGC CACTGCCCCC GCCAGAGCCC ACCGGCCAGC ATG TCC TCT GCT CAC 115

Met Ser Ser Ala His

                5

TTC AAC CGA GGC CCT GCC TAC GGG CTG TCA GCC GAG GTT AAG AAC AAG 163

Phe Asn Arg Gly Pro Ala Tyr Gly Leu Ser Ala Glu Val Lys Asn Lys

                10                  15                  20

CTG GCC CAG AAG TAT GAC CAC CAG CGG GAG CAG GAG CTG AGA GAG TGG 211

Leu Ala Gln Lys Tyr Asp His Gln Arg Glu Gln Glu Leu Arg Glu Trp

            25                  30                  35

ATC GAG GGG GTG ACA GGC CGT CGC ATC GGC AAC AAC TTC ATG GAC GGC 259

Ile Glu Gly Val Thr Gly Arg Arg Ile Gly Asn Asn Phe Met Asp Gly

        40                  45                  50

CTC AAA GAT GGC ATC ATT CTT TGC GAA TTC ATC AAT AAG CTG CAG CCA 307

Leu Lys Asp Gly Ile Ile Leu Cys Glu Phe Ile Asn Lys Leu Gln Pro

    55                  60                  65

GGC TCC GTG AAG AAG ATC AAT GAG TCA ACC CAA AAT TGG CAC CAG CTG 355

Gly Ser Val Lys Lys Ile Asn Glu Ser Thr Gln Asn Trp His Gln Leu

70                    75                    80                    85

GAG AAC ATC GGC AAC TTC ATC AAG GCC ATC ACC AAG TAT GGG GTG AAG     403

Glu Asn Ile Gly Asn Phe Ile Lys Ala Ile Thr Lys Tyr Gly Val Lys

                90                    95                   100

CCC CAC GAC ATT TTT GAG GCC AAC GAC CTG TTT GAG AAC ACC AAC CAT     451

Pro His Asp Ile Phe Glu Ala Asn Asp Leu Phe Glu Asn Thr Asn His

            105                   110                   115

ACA CAG GTG CAG TCC ACC CTC CTG GCT TTG GCC AGC ATG GCG AAG ACG     499

Thr Gln Val Gln Ser Thr Leu Leu Ala Leu Ala Ser Met Ala Lys Thr

        120                   125                   130

AAA GGA AAC AAG GTG AAC GTG GGA GTG AAG TAC GCA GAG AAG CAG GAG     547

Lys Gly Asn Lys Val Asn Val Gly Val Lys Tyr Ala Glu Lys Gln Glu

    135                   140                   145

CGG AAA TTC GAG CCG GGG AAG CTA AGA GAA GGG CGG AAC ATC ATT GGG     595

Arg Lys Phe Glu Pro Gly Lys Leu Arg Glu Gly Arg Asn Ile Ile Gly

150                   155                   160                   165

CTG CAG ATG GGC ACC AAC AAG TTT GCC AGC CAG CAG GGC ATG ACG GCC     643

Leu Gln Met Gly Thr Asn Lys Phe Ala Ser Gln Gln Gly Met Thr Ala

                170                   175                   180

TAT GGC ACC CGG CGC CAC CTC TAC GAC CCC AAG CTG GGC ACA GAC CAG     691

Tyr Gly Thr Arg Arg His Leu Tyr Asp Pro Lys Leu Gly Thr Asp Gln

            185                   190                   195

CCT CTG GAC CAG GCG ACC ATC AGC CTG CAG ATG GGC ACC AAC AAA GGA     739

Pro Leu Asp Gln Ala Thr Ile Ser Leu Gln Met Gly Thr Asn Lys Gly

        200                   205                   210

GCC AGC CAG GCT GGC ATG ACT GCG CCA GGG ACC AAG CGG CAG ATC TTC     787

Ala Ser Gln Ala Gly Met Thr Ala Pro Gly Thr Lys Arg Gln Ile Phe

    215                   220                   225

GAG CCG GGG CTG GGC ATG GAG CAC TGC GAC ACG CTC AAT GTC AGC CTG 835
Glu Pro Gly Leu Gly Met Glu His Cys Asp Thr Leu Asn Val Ser Leu
230                 235                 240                 245
CAG ATG GGC AGC AAC AAG GGC GCC TCG CAG CGG GGC ATG ACG GTG TAT 883
Gln Met Gly Ser Asn Lys Gly Ala Ser Gln Arg Gly Met Thr Val Tyr
                250                 255                 260
GGG CTG CCA CGC CAG GTC TAC GAC CCC AAG TAC TGT CTG ACT CCC GAG 931
Gly Leu Pro Arg Gln Val Tyr Asp Pro Lys Tyr Cys Leu Thr Pro Glu
            265                 270                 275
TAC CCA GAG CTG GGT GAG CCC GCC CAC AAC CAC CAC GCA CAC AAC TAC 979
Tyr Pro Glu Leu Gly Glu Pro Ala His Asn His His Ala His Asn Tyr
        280                 285                 290
TAC AAT TCC GCC TAGGGCCACA AGGCCTTCCC TGTTTTCCCC CCAAGGGAGG 1031
Tyr Asn Ser Ala
    295
CTGCTGCTGC TCTTGGCTGG ACCCAGCCAG GCCCAGCCGA CCCCCTCTCC CTGCATGGCA 1091
TCCTCCAGCC CCTGTAGAAC TCAACCTCTA CAGGGTTAGA GTTTGGAGAG AGCAGACTGG 1151
CGGGGGGCCC ATTGGGGGGA AGGGGACCCT CCGCTCTGTA GTGCTACAGG GTCCAACATA 1211
GAGCCGGGTG TCCCCAACAG CGCCCAAAGG ACGCACTGAG CAACGCTATT CCAGCTGTCC 1271
CCCCACTCCC TCACAAGTGG GTACCCCCAG GACCAGAAGC TCCCCCAGCA AAGCCCCCAG 1331
AGCCCAGGCT CGGCCTGCCC CCACCCCATT CCCGCAGTGG GAGCAAACTG CATGCCCAGA 1391
GACCCAGCGG ACACACGCGG TTTGGTTTGC AGCGACTGGC ATACTATGTG GATGTGACAG 1451
TGGCGTTTGT AATGAGAGCA CTTTCTTTTT TTTCTATTTC ACTGGAGCAC AATAAATGGC 1511
TGTAAAATCT C 1522

INFORMATION ON SEQ ID NO:2
(i) SEQUENCE CHARACTERISTICS:
(A) LENGTH: 297 amino acids

(B) TYPE: amino acid

(D) TOPOLOGY: linear

(ii) MOLECULE TYPE: protein

(xi) SEQUENCE DESCRIPTION: SEQ ID NO:2:

Met Ser Ser Ala His Phe Asn Arg Gly Pro Ala Tyr Gly Leu Ser

                5                   10                  15

Ala Glu Val Lys Asn Lys Leu Ala Gln Lys Tyr Asp His Gln Arg

                20                  25                  30

Glu Gln Glu Leu Arg Glu Trp Ile Glu Gly Val Thr Gly Arg Arg

                35                  40                  45

Ile Gly Asn Asn Phe Met Asp Gly Leu Lys Asp Gly Ile Ile Leu

                50                  55                  60

Cys Glu Phe Ile Asn Lys Leu Gln Pro Gly Ser Val Lys Lys Ile

                65                  70                  75

Asn Glu Ser Thr Gln Asn Trp His Gln Leu Glu Asn Ile Gly Asn

                80                  85                  90

Phe Ile Lys Ala Ile Thr Lys Tyr Gly Val Lys Pro His Asp Ile

                95                  100                 105

Phe Glu Ala Asn Asp Leu Phe Glu Asn Thr Asn His Thr Gln Val

                110                 115                 120

Gln Ser Thr Leu Leu Ala Leu Ala Ser Met Ala Lys Thr Lys Gly

                125                 130                 135

Asn Lys Val Asn Val Gly Val Lys Tyr Ala Glu Lys Gln Glu Arg

                140                 145                 150

Lys Phe Glu Pro Gly Lys Leu Arg Glu Gly Arg Asn Ile Ile Gly

                155                 160                 165

Leu Gln Met Gly Thr Asn Lys Phe Ala Ser Gln Gln Gly Met Thr

                170                 175                 180

Ala Tyr Gly Thr Arg Arg His Leu Tyr Asp Pro Lys Leu Gly Thr
                185                 190                 195
Asp Gln Pro Leu Asp Gln Ala Thr Ile Ser Leu Gln Met Gly Thr
                200                 205                 210
Asn Lys Gly Ala Ser Gln Ala Gly Met Thr Ala Pro Gly Thr Lys
                215                 220                 225
Arg Gln Ile Phe Glu Pro Gly Leu Gly Met Glu His Cys Asp Thr
                230                 235                 240
Leu Asn Val Ser Leu Gln Met Gly Ser Asn Lys Gly Ala Ser Gln
                245                 250                 255
Arg Gly Met Thr Val Tyr Gly Leu Pro Arg Gln Val Tyr Asp Pro
                260                 265                 270
Lys Tyr Cys Leu Thr Pro Glu Tyr Pro Glu Leu Gly Glu Pro Ala
                275                 280                 285
His Asn His His Ala His Asn Tyr Tyr Asn Ser Ala
                290                 295

INFORMATION ON SEQ ID NO:3
(i) SEQUENCE CHARACTERISTICS:
(A) LENGTH: 24 base pairs
(B) TYPE: nucleic acid
(C) STRANDNESS: single
(D) TOPOLOGY: linear
(ii) MOLECULE TYPE: Other nucleic acids synthetic DNA
(xi) SEQUENCE DESCRIPTION: SEQ ID NO:3:
GAGTGTGCAG ACGGAACTTC AGCC

INFORMATION ON SEQ ID NO:4

(i) SEQUENCE CHARACTERISTICS:
(A) LENGTH: 24 base pairs
(B) TYPE: nucleic acid
(C) STRANDNESS: single
(D) TOPOLOGY: linear
(ii) MOLECULE TYPE: Other nucleic acids synthetic DNA
(xi) SEQUENCE DESCRIPTION: SEQ ID NO:4:
GGCTGGGCCT GGCTGGGTCC AGCC

## Claims

1. A therapeutic agent for arteriosclerosis, which comprises a calponin gene as an active ingredient.

2. The therapeutic agent for arteriosclerosis of claim 1, wherein said calponin gene contains a base sequence encoding the amino acid sequence of SEQ ID No: 2.

3. The therapeutic agent for arteriosclerosis of claim 1, wherein said calponin gene contains the base sequence of SEQ ID No: 1.

4. The therapeutic agent for arteriosclerosis of any one of claims 1-3, wherein said calponin gene is encapsulated in a synthetic or naturally occurring membrane.

5. The therapeutic agent for arteriosclerosis of claim 4, wherein said calponin gene is encapsulated in liposome.

6. The therapeutic agent for arteriosclerosis of any one of claims 1-3, wherein said calponin gene is contained in a recombinant vector that is capable of expressing the calponin gene in a smooth muscle cell.

7. The therapeutic agent for arteriosclerosis of claim 6, wherein said recombinant vector contains a promoter.

8. The therapeutic agent for arteriosclerosis of claim 7, wherein said promoter is a β-actin promoter.

9. The therapeutic agent for arteriosclerosis of claim 8, wherein said recombinant vector is recombinant vector pCAGGS/hCN in a size of about 6.5 kb that contains an enhancer of cytomegalovirus, chicken β-actin promoter, rabbit β-globin 3' splice signal, rabbit β-globin 3'-flanking region, SV40 replication origin and human calponin cDNA and which is represented by the following restriction map.

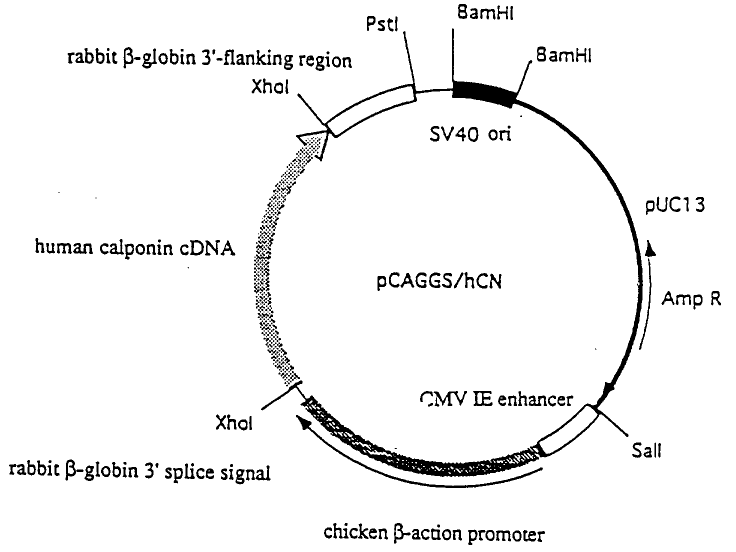
PstI
8amHI
8amHI
rabbit β-globin 3'-flanking region
XhoI
SV40 ori
pUC13
human calponin cDNA
pCAGGS/hCN
Amp R
CMV IE enhancer
XhoI
SalI
rabbit β-globin 3' splice signal
chicken β-action promoter

10. The therapeutic agent for arteriosclerosis of any one of claims 6-9, wherein said recombinant vector containing the calponin gene is encapsulated in a synthetic or naturally occurring membrane.

11. The therapeutic agent for arteriosclerosis of claim 10, wherein said recombinant vector containing the calponin gene is encapsulated in liposome.

12. A recombinant vector that contains a calponin gene and which is capable of expressing the calponin gene in a smooth muscle cell.

13. The recombinant vector of claim 12, wherein said calponin gene contains a base sequence encoding the amino acid sequence of SEQ ID No: 2.

14. The recombinant vector of claim 13, wherein said calponin gene contains the base sequence of SEQ ID No: 1.

15. The recombinant vector of any one of claims 12-14 which contains a promoter.

16. The recombinant vector of claim 15, wherein said promoter is a β-actin promoter.

17. The recombinant vector of claim 16 which is recombinant vector pCAGGS/hCN in a size of about 6.5 kb that contains an enhancer of cytomegalovirus, chicken β-actin promoter, rabbit β-globin 3' splice signal, rabbit β-globin 3'-flanking region, SV40 replication origin and human calponin cDNA and which is represented by the following restriction map.

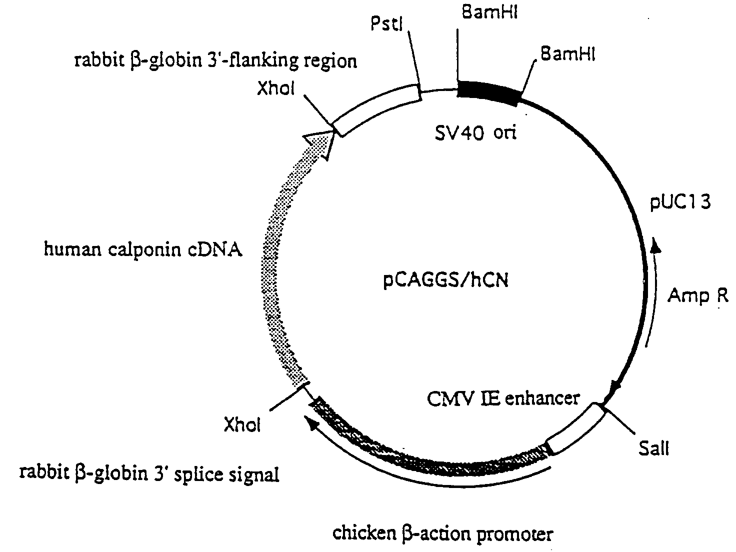
PstI
BamHI
BamHI
rabbit β-globin 3'-flanking region
XhoI
SV40 ori
pUC13
human calponin cDNA
pCAGGS/hCN
Amp R
CMV IE enhancer
XhoI
SalI
rabbit β-globin 3' splice signal
chicken β-action promoter

18. A pharmaceutical composition comprising a calponin gene as an active ingredient and a pharmaceutically acceptable carrier.

19. The pharmaceutical composition of claim 18 which is used for treating arteriosclerosis.

20. The pharmaceutical composition of claim 18 which is used for treating an ischemic heart disease.

21. The pharmaceutical composition of claim 20, wherein said ischemic heart disease is angina pectoris.

22. The pharmaceutical composition ofd claim 20, wherein said ischemic heart disease is cardiac infarction.

23. The use of a therapeutically effective amount of a calponin gene for the preparation of a pharmaceutical.

24. The use according to claim 23, wherein the pharmaceutical is effective in the treatment of ischemic heart disease.

25. The use according to claim 24, wherein said ischemic heart disease is angina pectoris.

26. The use according to claim 24, wherein said ischemic heart disease is cardiac infarction.
